(19) Europäisches Patentamt
European Patent Office
Office européen des brevets

(11) Publication number: **0 075 047**
**A1**

# (12) EUROPEAN PATENT APPLICATION

(21) Application number: **81304274.4**

(22) Date of filing: **17.09.81**

(51) Int. Cl.³: **C 07 C 157/09**, C 07 D 295/18, A 61 K 31/17, A 61 K 31/445

(43) Date of publication of application: **30.03.83 Bulletin 83/13**

(84) Designated Contracting States: **AT BE CH DE FR GB IT LI LU NL SE**

(71) Applicant: **STAUFFER CHEMICAL COMPANY, Westport Connecticut 06880 (US)**

(72) Inventor: **Pallos, Ferenc M., 136 Twin Peaks Drive, Walnut Creek California 94598 (US)**
Inventor: **Teach, Eugene Gordon, 1929 Downey Place, El Cerrito California 94530 (US)**
Inventor: **DeBaun, Jack Rollie, 1111 Lorne Way, Sunnyvale California 94087 (US)**

(74) Representative: **Smith, Sydney et al, Elkington and Fife High Holborn House 52/54 High Holborn, London WC1V 6SH (GB)**

## (54) 1-(Substituted phenylthiocarbamyl) 3,3-disubstituted formamidines and their production and pharmaceutical compositions containing them.

(57) 1-(substituted phenylthiocarbamyl) 3,3-disubstituted formamidine compounds having the structural formula

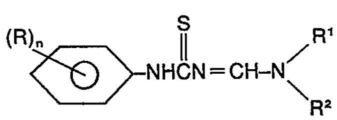

wherein

n is 1, 2 or 3,

R is alkyl having 1 to 6 carbon atoms, alkoxy having 1 to 6 carbon atoms, alkylthio having 1 to 4 carbon atoms, halogen, nitro, acyl having 1 to 4 carbon atoms, hydroxyl and

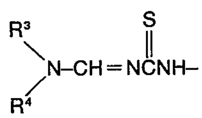

wherein $R^3$ and $R^4$ have the same meaning as $R^1$ and $R^2$ defined below,

$R^1$ and $R^2$ are the same or different and are alkyl having 1 to 6 carbon atoms, or taken together represent the chain $-(CH_2)_p-(X)_m-(CH_2)_q$ and forming a ring structure with the nitrogen to which they are bound wherein p is 1, 2 or 3, q is 2 or 3, X represents $-O-$, $-S-$, $-SO-$, $-SO_2-$, $-NR^3-$, $R^3$ is hydrogen or alkyl having 1 to 6 carbon atoms, and m is 0 or 1 and salts thereof have anti-inflammatory, anti-oedemic and anti-hypertensive activity.

They are prepared by the reaction of the parent thiourea and dialkyl acetal.

EP 0 075 047 A1

Title

1-(Substituted phenylthiocarbamyl)3,3-disubstituted formamidines and their production and pharmaceutical compositions containing them.

This invention relates to 1-(substituted phenylthiocarbamyl) 3,3-disubstituted formamidines, to processes for their production, to pharmaceutical compositions containing them and to their use in pharmacy.

In U.S. 3,959,368 there is disclosed the compound N,N-dimethyl-N'-phenylthiocarbamyl formamidine and its use as an anti-inflammatory.

We have found that compounds of the following structural formula:

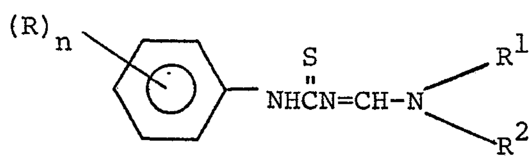

I

wherein

n is the integer 1, 2 or 3, preferably 1 or 2;

R is alkyl having 1 to 6 carbon atoms, preferably 1 to 4 carbon atoms, more preferably methyl; alkoxy having 1 to 6 carbon atoms, preferably 1 to 4 carbon atoms, more preferably methoxy; alkylthio having 1 to 4 carbon atoms, preferably methylthio; halogen, preferably chlorine; nitro; acyl having 1 to 4 carbon atoms, preferably acetyl; hydroxyl and

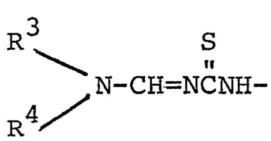

wherein $R^3$ and $R^4$ have the same meaning as $R^1$ and $R^2$ defined below;

$R^1$ and $R^2$ are the same or different and are alkyl having 1 to 6 carbon atoms, preferably 1 to 3 carbon atoms, more preferably methyl or taken together represent the chain $-(CH_2)_p-(X)_m-(CH_2)_q$ and forming a ring structure with the nitrogen to which they are bound wherein p is 1, 2 or 3, q is 2 or 3, X represents -O-, -S-, -SO-, $-SO_2-$, $-NR^3-$, $R^3$ is hydrogen or alkyl having 1 to 6 carbon atoms, preferably methyl, and m is 0 or 1.

The invention therefore provides compounds of the above general formula either as such or in the form of salts thereof, in particular non-toxic pharmaceutically acceptable salts, such as salts with organic and inorganic acids have activity, showing anti-inflammatory, anti-oedema and anti-hypertensive activity.

The compounds of this invention can be used for their intended pharmaceutical purposes either as the free base (described above) or in the form of suitable organic or inorganic acid salts. A preferred form is the hydrochloric acid salt.

The compounds according to the invention may be prepared by reaction of a thiourea II, with a dialkyl acetal III,

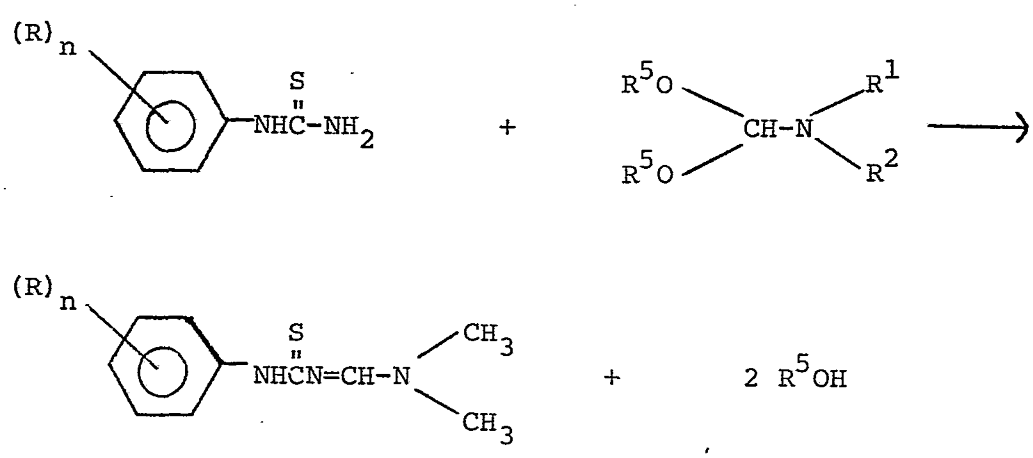

in which R, $R^1$, $R^2$ and n are as defined above and $R^5$ represents an alkyl group preferably containing 1 to 6 carbon atoms, in particular methyl.

The compounds may be isolated as the free base or in salt form, as desired.

Generally, the above reaction may be carried out by dissolving the two reactants in a solvent such as benzene or toluene, heating the solution at reflux for about 15 minutes, slowly distilling off the solvent-alkanol mixture in particular solvent-methanol mixture when $R^5$ is methyl, cooling the undistilled solution to room temperature and the recovering of the desired product by filtration. The recovered product can be purified by standard procedures.

The following examples illustrate the process for the preparation of the compounds:-

Example I

1-(p-methoxyphenylthiocarbamyl) 3,3-dimethyl formamidine hydrochloride

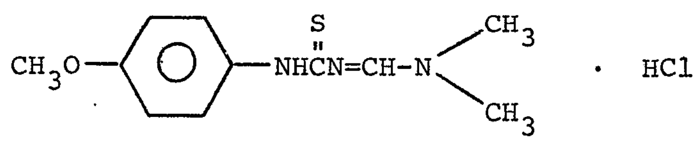

7.3 grams (g) p-methoxyphenylthiourea and 5.0g dimethylformamide dimethyl acetal and 100 milliliters (ml) benzene were placed in a reaction flask equipped with a distillation head. The mixture was gently refluxed for 15 minutes with the slow removal of the benzene methanol azeotrope mixture through the distillation head. After cooling, the formed 8.0 g precipitate was recovered by filtration with a melting point (m.p.) 140-142°C. The filtrate was treated with a cold ether/HCl mixture and 1.0 g precipitated hydrochloride filtrate was recovered with a m.p. 150°C with decomposition (dec.).

The following compounds were prepared by the same procedure using the appropriate starting materials. Compound numbers are assigned to each compound and are used throughout the remainder of the specification.

Table 1

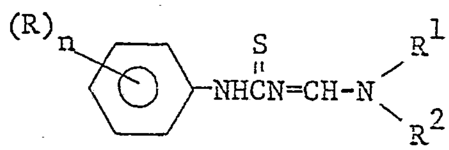

· HCl

| Compound Number | n | R | $R^1$ | $R^2$ | m.p. °C |
|---|---|---|---|---|---|
| 1 | 1 | $4-CH_3O$ | $CH_3$ | $CH_3$ | 150 dec. |
| 2 | 1 | 4-Cl | $CH_3$ | $CH_3$ | 170 dec. |
| 3 | 2 | $2,6-C_2H_5$ | $CH_3$ | $CH_3$ | 167-8 dec. |
| 4 | 2 | $2,4-CH_3$ | $CH_3$ | $CH_3$ | 170-1 dec. |
| 5 | 1 | $4-NO_2$ | $CH_3$ | $CH_3$ | 169-174° |
| 6 | 1 | $4-CH_3\underset{O}{\underset{\|}{C}}$ | $CH_3$ | $CH_3$ | 174-176° |
| 7 | 1 | 4-HO | $CH_3$ | $CH_3$ | |
| 8 | 2 | $3,4-CH_3$ | $CH_3$ | $CH_3$ | 179-181° |
| 9 | 2 | $3,4-Cl_2$ | $CH_3$ | $CH_3$ | 176-179° |

| Compound Number | n | R | $R^1$ | $R^2$ | m.p. °C |
|---|---|---|---|---|---|
| 10 | 1 | 3-Cl | $CH_3$ | $CH_3$ | 173-177° |
| 11 | 1 | 2-Cl | $CH_3$ | $CH_3$ | 180-183° |
| 12 | 2 | 2,5-$CH_3O$ | $CH_3$ | $CH_3$ | 175-178° |
| 13 | 1 | 4-$C_2H_5$ | $CH_3$ | $CH_3$ | 182-184° |
| 14 | 2 | 2,6-$CH_3$ | $CH_3$ | $CH_3$ | 172-175° |
| 15 | 2 | 2,6-$Cl_2$ | $CH_3$ | $CH_3$ | 177-180° |
| 16 | 2 | 2,4-$Cl_2$ | $CH_3$ | $CH_3$ | 180-182° |
| 17 | 1 | 2-$CH_3O$ | $CH_3$ | $CH_3$ | 173-175° |
| 18 | 1 | 3-$CH_3O$ | $CH_3$ | $CH_3$ | 163-166° |
| 19 | 2 | 2,4-$CH_3$ | $-CH_2CH_2CH_2CH_2CH_2-$ | | 145-151° |
| 20 | 1 | * | $CH_3$ | $CH_3$ | 190-193° |

* 3- 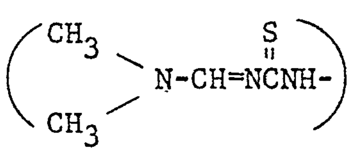

## Anti-Inflammatory Screening

Anti-inflammatory activity for a compound is demonstrated by either one of two tests. The first test is for adjuvant arthritis inhibition and the second, an anti-edema test, involves the diminution of experimental edema induced in the hind paw of a rat by the injection of carrageenin.

### Adjuvant Arthritis Inhibition

This test was run according to the test procedure recited by VanArman, G. C., Nuss, G. W. and Risley, E. A., J. Pharmacol. Exper. Terap., 187:400-413, 1973. The following is a description of the test:

BAD ORIGINAL

The hind-foot volumes of five Wistar male rats (Taiwan strain) weighing 130-160 g each were measured by fluid displacement immediately prior to intraplantar administration (right hind paw) of 0.1 ml of an adjuvant suspension containing 5 milligrams (mg) Mycobacterium butyricum bacilli suspended in heavy mineral oil. Test compounds were orally administered to the animals for five days beginning the day before injection of the adjuvant. Four hours after the fifth daily drug dose the volume of the right hind paw (primary lesion) was measured for determination of any acute anti-inflammatory effect. Fourteen days after adjuvant inoculation the volume of the uninjected foot was measured (secondary lesion) for determination of prolonged anti-inflammatory effect or immunosuppressant effect. The percent reduction in foot volume was calculated for each hind paw in relation to corresponding control values determined simultaneously. A greater than 30 percent reduction in volume of either paw is considered a significant effect. Reduction of the primary lesion without effect on the secondary lesion denotes acute anti-inflammatory activity of short to moderate duration. Reduction of the secondary lesion without effect on the primary lesion suggests interference with the immunological component of polyarthritis disease. Reduction of both lesions suggests anti-inflammatory activity of long duration or a mixture of anti-inflammatory and immunosuppressant effects as occurs with corticosteroids.

Table 2 shows the reduction in edema of the primary and secondary lesions according to the above-described test procedure at the indicated rate.

BAD ORIGINAL

Table 2

Percent Reduction in Edema of the Primary and Secondary Lesions at Rate Shown

| Compound Number | Rate mg/kg | Primary | Secondary |
|---|---|---|---|
| 1 | 5 | 39% | 35% |
| 2 | * | | |
| 3 | 100 | 39% | 31% |
| 4 | 100 | 0% | 0% |

*Not tested

Anti-edema

Anti-edema effect was determined according to the test procedure recited by Winter, C.A., Risley, E. A., and Nuss, G. W., Proc. Exper. Biol. Med. 111:544-547, 1962. For this test, three male Wistar rats (Taiwan strain) weighing 100-120 g each are orally dosed with test compound dissolved or suspended in 3 ml of water. One hour later, the plantar surface of the right hind paw was injected with 0.1 ml of a 1 percent suspension of carrigeenan in saline and the left paw was similarly injected with saline only. Three hours after the injection the volume of both hind paw was measured by fluid displacement and the percent decrease in carrigeenan-induced swelling (volume of carrigeenan injected foot minus saline injected foot) was determined by comparison with untreated (no test compound) animals. Greater than 30 percent inhibition is considered evidence of an anti-edema effect.

BAD ORIGINAL

Table 3 shows the reduction in edema in the hind paw of a rat according to the above-described test procedure at the rate indicated.

Table 3

Percent Reduction in Edema

| Compound Number | Rate mg/kg | Reduction of Induced Edema |
|---|---|---|
| 1 | 5 | 35% |
| 2 | 25 | 32% |
| 3 | 100 | 33% |
| 4 | * | |
| phenylbutazone (standard) | 100 | 41% |

*Not tested

Anti-Hypertensive Screening

This test was run according to the test procedure recited by Nagoaka, A., Kikuchi, K. and Aramaki, Y., Jap. J. Pharmacol., 19:401-408, 1969. For this test, two normotensive Wistar rats (Taiwan strain) were placed in perspex holders and then placed in a heated chamber at 40°C for 4-6 minutes before treatment. They were maintained there throughout each experiment. Systolic blood pressure was recorded by indirect means employing a tail cuff and pneumatic sensor device applied to the base of the animals' tails. The device was coupled to a programmed Narco-Biosystems sphygmomano meter (Model PE-300), an instrument for measuring blood pressure, and

Sanei polygraph. After recording suitably steady blood pressure readings, test drugs were orally administered and blood pressure checked again 2, 4 and 6 hours after drug administration. A mean decrease of more than 10 percent from the pretreatment value at any two consecutive measurement intervals is considered evidence of hypotensive effect.

The results of the anti-hypertensive test are reported in Table 4.

Table 4

Percent Mean Decrease in Systolic Blood Pressure

| Compound Number | Rate mg/kg | 2 hours | 4 hours | 6 hours |
|---|---|---|---|---|
| 1-3 | * | | | |
| 4 | 100 | 4% | 17% | 15% |
| α-methyl 3,4-dihydroxy-phenylalanine (standard) | 100 | 11% | 21% | 19% |

*decreases of less than 10% were observed for compounds 1-3 and are not reported

The compounds of the present invention, either alone or in the form of pharmaceutical composition may be administered to an animal subject in any of a number of forms and via any of several routes. Thus, the compounds or compositions thereof may be orally administered in the form of tablets, pills, capsules, or in the form of a suspension. The compounds may also be administered parenterally in the form of an injectable solution or suspension. The compounds or compositions thereof may also be administered topically, in the form of an ointment or rectally, in the form of a suppository.

When orally administering the compounds or compositions, use can be made of a tablet, pill or capsule consisting entirely of the desired compound, although ordinarily, a composition comprising an effective amount of the compound and varying amounts of one or more physiologically inert materials such as carriers, vehicles, binders and the like will be used. Additionally, the compounds may be orally administered in the form of a suspension thereof in a suitable vehicle such as a syrup.

When parenterally administering the compounds or compositions, use may be made of a parenteral solution or suspension of the compound in a suitable solvent or suspension medium.

The compounds of the present invention may also be administered rectally in the form of a suppository comprising an effective amount of the desired compound and a suitable vehicle such as petroleum jelly.

Claims:

1. Compounds of the general formula:-

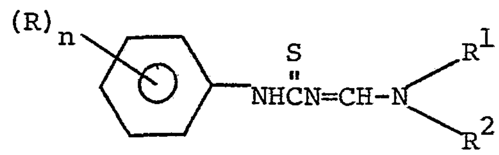 I

in which

n is the integer 1, 2 or 3, R is alkyl having 1 to 6 carbon atoms, alkoxy having 1 to 6 carbon atoms, alkylthio having 1 to 4 carbon atoms, halogen, nitro, acyl having 1 to 4 carbon atoms, hydroxyl and

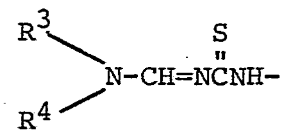 wherein $R^3$ and $R^4$ have the same meaning as $R^1$ and $R^2$ defined below;

$R^1$ and $R^2$ are the same or different and are alkyl having 1 to 6 carbon atoms, or taken together represent the chain $-(CH_2)_p-(X)_m-(CH_2)_q$ and forming a ring structure with the nitrogen to which they are bound wherein p is 1, 2 or 3, q is 2 or 3, X represents -O-, -S-, -SO-, $-SO_2-$, $-NR^3-$, $R^3$ is hydrogen or alkyl having 1 to 6 carbon atoms, and m is 0 or 1; and salts thereof.

2. A compound as claimed in claim 1 characterised in that n is 1 R is 4-methoxy, $R^1$ is methyl and $R^2$ is methyl.

3. A compound as claimed in claim 1 characterised in that n is 1 R is 4-chlorine, $R^1$ is methyl and $R^2$ is methyl.

4. A compound as claimed in claim 1 characterised in that n is 2 R is 2,6-diethyl, $R^1$ is methyl and $R^2$ is methyl.

5. A compound as claimed in claim 1 characterised in that n is 2, R is 2,4-dimethyl, $R^1$ is methyl and $R^2$ is methyl.

6. Compounds as claimed in claim 1 in the form of salts with suitable organic or inorganic acid.

7. A process for the preparation of compounds as claimed in claim 1 which comprises reacting a thiourea of the general formula II

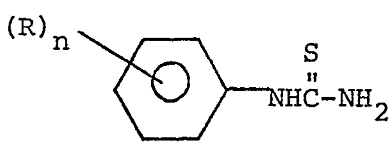

in which R has the meaning given in claim 1 with a dialkyl acetal of the formula III

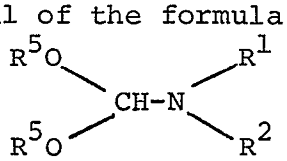

in which $R^5$ represents an alkyl group and $R^1$ and $R^2$ have the meanings given above, the product if desired being recovered as a salt or converted to such salt.

8. A process as claimed in claim 7 in which $R^5$ is $CH_3$.

9. A pharmaceutical composition characterised in that it contains a compound as claimed in claim 1 in association with an inert pharmaceutical carrier or diluent.

Claims for Austria:

1. A process for the preparation of compounds of the general formula

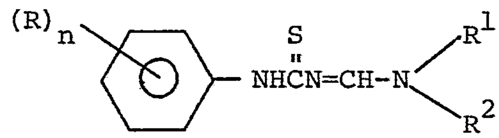

I

in which

n is the integer 1, 2 or 3, R is alkyl having 1 to 6 carbon atoms, alkoxy having 1 to 6 carbon atoms, alkylthio having 1 to 4 carbon atoms, halogen, nitro, acyl having 1 to 4 carbon atoms, hydroxyl and

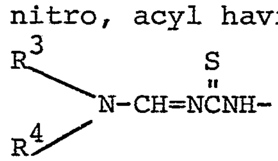

wherein $R^3$ and $R^4$ have the same meaning as $R^1$ and $R^2$ defined below;

$R^1$ and $R^2$ are the same or different and are alkyl having 1 to 6 carbon atoms, or taken together represent the chain $-(CH_2)_p-(X)_m-(CH_2)_q$ and forming a ring structure with the nitrogen to which they are bound wherein p is 1, 2 or 3, q is 2 or 3, X represents -O-, -S-, -SO-, $-SO_2-$, $-NR^3-$, $R^3$ is hydrogen or alkyl having 1 to 6 carbon atoms, and m is 0 or 1; and salts thereof;

which comprises reacting a thiourea of the general formula II

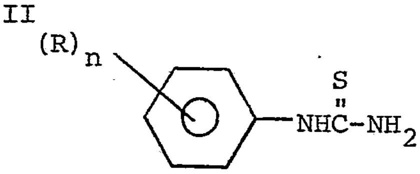

in which R has the meaning given above with a dialkyl acetal of the formula III

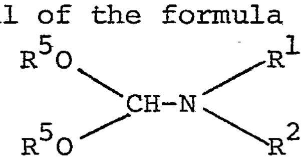

in which $R^5$ represents an alkyl group and $R^1$ and $R^2$ have the meanings given above, the product if desired being recovered as a salt or converted to such salt.

2. A process as claimed in claim 1 characterised in that dialkyl acetal is used in which $R^5$ is methyl.

3. A process as claimed in claim 1 for the preparation of 1-(p-methoxyphenylthiocarbamyl) 3,3-dimethyl formamidine hydrochloride which comprises reacting p-methoxphenylthiourea with dimethylformamide dimethyl acetal and recovering the product as the hydrochloride.

0075047

European Patent Office

# EUROPEAN SEARCH REPORT

Application number
EP 81 30 4274

## DOCUMENTS CONSIDERED TO BE RELEVANT

| Category | Citation of document with indication, where appropriate, of relevant passages | Relevant to claim |
|---|---|---|
| X | JOURNAL OF ORGANIC CHEMISTRY, vol. 45, no.19, September 12 1980 WASHINGTON DC (US)<br>YANG-I LIN et al.: "New Synthesis of 1,2,4-Thiadiazoles"<br>pages 3750-3753<br>* Page 3751, table 1, compounds 2j, 2k,21,2m; page 3752, experimental section * | 1,7,8 |
| | -- | |
| DY | US - A - 3 959 368 (JACK R. DeBAUN)<br>* The whole document * | 1,7-9 |
| | -- | |
| Y | US - A - 4 012 527 (JACK R. DeBAUN)<br>* The whole document * | 1,6-9 |
| | ---- | |

**CLASSIFICATION OF THE APPLICATION (Int. Cl. 3)**

C 07 C 157/09
C 07 D 295/18
A 61 K 31/17
A 61 K 31/445

**TECHNICAL FIELDS SEARCHED (Int.Cl. 3)**

C 07 C 157/00
C 07 D 295/00

**CATEGORY OF CITED DOCUMENTS**

X: particularly relevant if taken alone
Y: particularly relevant if combined with another document of the same category
A: technological background
O: non-written disclosure
P. intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 28-04-1982 | HENRY |